Europäisches Patentamt

European Patent Office

Office européen des brevets

(19)

(11) Publication number: **0 189 331 B1**

## EUROPEAN PATENT SPECIFICATION

(12)

(45) Date of publication of patent specification: **16.10.91**   (51) Int. Cl.⁵: **G02C 7/16, A61F 9/04**

(21) Application number: **86300499.0**

(22) Date of filing: **24.01.86**

(54) **Objective material.**

(30) Priority: **24.01.85 JP 9849/85**
**15.02.85 JP 26473/85**
**28.02.85 JP 27244/85 U**

(43) Date of publication of application:
**30.07.86 Bulletin 86/31**

(45) Publication of the grant of the patent:
**16.10.91 Bulletin 91/42**

(84) Designated Contracting States:
**DE FR GB IT**

(56) References cited:
| | |
|---|---|
| EP-A- 0 141 736 | BE-A- 775 442 |
| GB-A- 1 026 839 | US-A- 2 230 009 |
| US-A- 3 731 993 | US-A- 3 967 885 |
| US-A- 4 249 803 | |

(73) Proprietor: **Kabushiki Kaisha Shuho**
**2010 Miyuki-4-chome**
**Fukui-shi Fukui-ken(JP)**

(72) Inventor: **Muraoka, Koji**
**33-4 Kamitonokuchicho**
**Sabae-shi Fukui-ken(JP)**
Inventor: **Tatibana, Fumiki**
**414 Nagamotocho**
**Fukui-shi Fukui-ken(JP)**
Inventor: **Nakatsuka, Katsuhiko**
**2-18 Omiya-3-chome**
**Fukui-shi Fukui-ken(JP)**
Inventor: **Niwa, Hiroyuki**
**66-2-1 Nishibukurocho**
**Sabae-shi Fukui-ken(JP)**
Inventor: **Yamatani, Tadahiro**
**13-5 Bunkyo-2-chome**
**Fukui-shi Fukui-ken(JP)**

(74) Representative: **Rees, David Christopher et al**
**Kilburn & Strode 30 John Street**
**London WC1N 2DD(GB)**

## Description

The present invention relates to an optical sheet material capable of screening ultraviolet rays and correcting such defects of vision as nearsightedness, farsightedness, astigmatism and presbyopia.

The incidence of ultraviolet rays on the eyes is generally prevented by the use of sunglasses made by provided parallel-surface glasses or plastics pieces with suitable light screening properties and/or absorbing properties, with respect to specific wavelengths, by adding a light-screening or absorbing substance during their raw material manufacturing stage, or colouring the transparent glasses or applying a coating to the surface of the glasses. In the case of a wearer having nearsightedness, farsightedness, astigmatism or presbyopia, glass or plastics lenses are employed to form spectacles, sometimes in combination with the above screening or absorbing properties. It is also quite common for ordinary spectacles to be used on top of or beneath sunglasses.

However, such sunglasses tend to be expensive and also, since they are not used continuously, if they are not to be damaged they require the provision of a case for carrying or accommodating the glasses. Also, in everyday life, particularly to obtain protection from intense sunshine in the summer, or the sunshine on a snowy day or the reflected light of such sunshine, it is often necessary to use a more powerful ultraviolet screening substitute in place of the sunglasses, and the same applies to any person having nearsightedness, farsightedness, astigmatism or presbyopia.

US 3967885 discloses an opaque mask attached to spectacle frames, the mask having a series of transparent areas. The arrangement is for post-operative cataract patients and provides protection against excessive light while improving the focusing ability of the eye.

EP-A-141736 (which constitutes a state of the art under Art.S4 (3) EPC) discloses a universal vision correction device in which a thin generally reflective sheet is placed over spectacle lenses. The sheet has small transparent zones.

It is the primary object of the present invention to provide an observation optical sheet material which is so designed that, where an extremely bright situation is suddenly experienced, and sunglasses and/or correction glasses are not to hand, it is nevertheless possible to observe such bright situation whilst protecting one's eyes by the use of a material according to the invention incorporated in one of those things kept at one's side or usually carried on the person, e.g. an item of stationery such as an underlaying sheet or ruler, or other commonplace item such as a book cover, a bookmarker, a corner of a book cover, a cash card, credit card or member's card, a watch band, a shoulder belt for bags or a tag.

In accordance with the invention there is provided an optical sheet material which is capable of permitting sufficient light transmission therethrough to support human vision, at least a part of the sheet being generally opaque but with a large number of spot-like transparent portions distributed uniformly thereover, characterised in that each spot-like transparent portion is formed of substantially hexagonal shape inscribed in or circumscribing a circle having a diameter between 0.7 and 2.0 mm, the sides of the hexagonal shape being curved arcuately inwards, the transparent portions being arranged in a staggered manner such that lines connecting their central points form regular triangles.

The transparent portions are distributed uniformly in a staggered manner, for example, so that the centres are spaced apart by between 2.0 to 5.5 mm. Suitable materials for the above-constructed optical material of this invention must be substances having a suitable strength, physical properties, elegance and durability required for use as the materials for the previously mentioned articles as well as a capacity for maintaining the transparency of the transparent portions for a comparatively long period of time, and these substances include for example plastics such as soft or hard polyvinyl chloride, polyacrylic, methacrylic acid, polyurethane, polyolefin and polyester plastics. It is to be noted that the transparent portions may take the form of holes made through the material and the material may be an opaque material such as a metal sheet. The invention is not restricted to the above-mentioned plastics.

On the other hand, the spot-like transparent portions and the opaque portion may be formed separately from each other may e.g. by a method in which an opaque portion is printed by photogravure on the surface of a transparent sheet of such plastics material so as to leave spot-like blank portions, and then a transparent film is applied onto the printed surface, or it may be a method of printing on opaque layer over the whole surface of a transparent film, and forming transparent portions or holes by such means as etching or punching. Also, any of various other methods may be used.

The thus constructed observation optical sheet material of this invention can be used daily in conformity with the purpose for which the article is primarily intended, so that when a need suddenly occurs to study an object or scene in a place where ultraviolet rays are abundant, whether indoors or outdoors, the optical material is held in front of the eyes and the object or scene is studied through it. In such a case, the amount of the incident light on the eyes through the spot-like transparent portions is restricted considerably by the opaque portions. As a result, movements take place subconsciously in the eyes to distinguish the object or scene in that the ciliary body (or the structure for adjusting the curvature of the crystalline lens in front of the eyeball) of each eye functions naturally in such a manner that the curvature of the lens is decreased if the person is nearsighted and the curvature is increased if the person is farsighted.

Thus, not only a person of normal vision but also a person having nearsightedness, farsightedness, astigmatism or presbyopia can comparatively accurately look at an object through the optical material according to the invention.

This principle is similar to that of a pinhole camera which takes a picture through a minute aperture,so that even if there are variations in the relative distance of an object corresponding to the object to be photographed in the case of a camera, the focal point is not varied much, owing to the long depth of focus, and therefore the object can be seen clearly through the optical material.

According to another aspect of the present invention, a dual function assembly comprises a readily portable and/or accessible object having a primary function, and also having, as a part of it, or associated with it, a piece of sheet material at least a portion of which has the secondary function of forming an optical device which is capable of permitting sufficient light transmission therethrough to support human vision, at least a part of the sheet being generally opaque but with a large number of spot-like transparent portions distributed uniformly thereover, wherein each spot-like transparent portion is formed of substantially hexagonal shape inscribing or circumscribing a circle having a diameter between 0.7 and 2.0 mm, the sides of the hexagonal shape being curved arcuately inwards, the transparent portions being arranged in a staggered manner such that lines connecting their central points form regular triangles.

The above and other objects as well as advantageous features of the invention will become more clear from the following description of certain specific embodiments taken in conjunction with the drawings, in which:-

Figure 1 is a partial plan view showing, in enlarged form, an embodiment of an optical material according to the invention;

Figures 2A, 2B and 2C show some exemplary geometrical shapes of the spot-like transparent portions though Figures 2B and 2C are outside the scope of the present invention;

Figure 3 is a graph showing the relation between the hole diameter and the eyesight (directional eyesight) or the definition of an object viewed through the holes formed in the optical material of Figure 1;

Figure 4 is a plan view of a watch band made of the optical material shown in Figure 1;

Figure 5 is a plan view of a book cover incorporating, in corner thereof, the optical material shown in Figure 1;

Figure 6 is a plan view of a bookmarker incorporating, in a part thereof, the optical material shown in Figure 1; and

Figure 7 shows an example in which the optical material of Figure 1 is incorporated in a cash card.

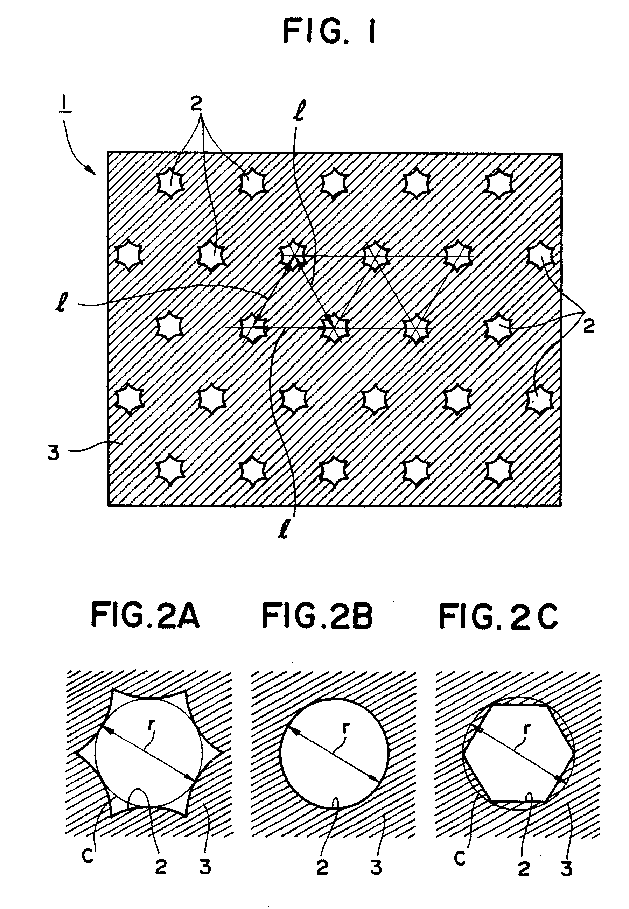

Referring to Figures 1 and 2A, numeral 1 designates an optical material according to the invention, and 2 a plurality of spot-like transparent portions each formed into a hexagonal shape such as one whose sides circumscribe a circle of a diameter r and are arcuately curved inwards. (The circular shape of a diameter r as shown in Figure 2B or a polygonal shape inscribing a circle of a diameter r as shown in Figure 2C are outside the scope of the present invention.) A plurality of such spot-like transparent portions 2 are each arranged at the apex of each of a plurality of contiguous regular triangles whose sides have a length of 2.0 to 5.5 mm, as shown at ℓ in Figure 1, and thus the transparent portions 2 are arranged in a staggered manner. Numeral 3 designates an opaque portion.

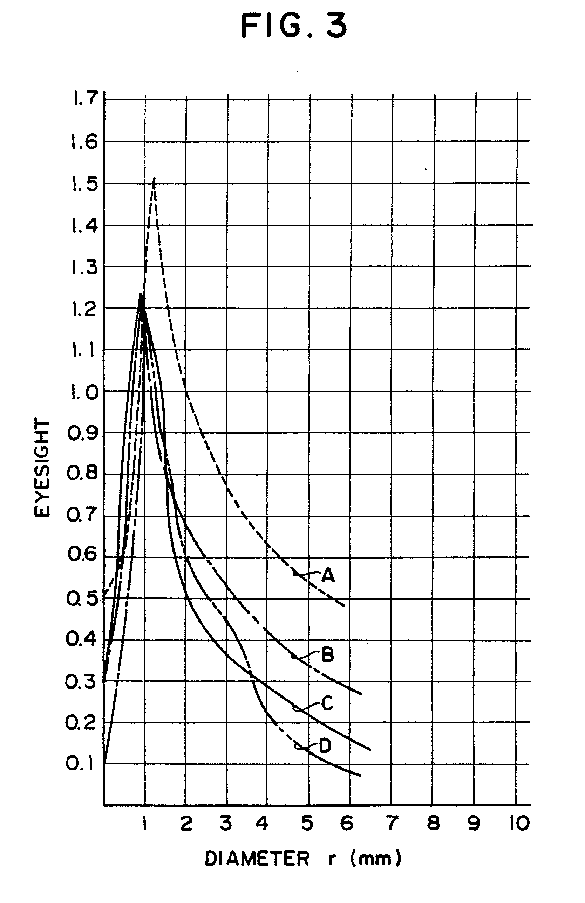

Referring to Figure 3, there is illustrated a graph showing the relation between the diameter r of the spot-like transparent portions 2 and the eyesight (directional eyesight) when looking at an object by using the optical material according to the embodiment of Figure 1. From this figure it will be seen that an essential requirement of the invention resides in that the diameter r of the plurality of spot-like transparent portions is selected between 0.7 and 2.0 mm and preferably between 1.0 and 1.2 mm.

It is to be noted that the spot-like transparent portions may take the form of spot-like holes formed through the material, and it has been confirmed that from the stand-point of reducing the interference in the field of view, it is preferable to arrange the transparent portions 2 uniformly at such positions that the distance between their centres is between 2.0 and 5.5 mm, that is, they are distributed so as to form regular triangles. A preferred range of the length of these sides is between 3.0 and 5.0 mm.

While it is more desirable to use a plastics material for forming the optical material 1 in the light of its physical properties, working properties during manufacture, cost and elegancy as mentioned previously, the invention is not necessarily limited to such plastics materials and the use of any material having the same effects is of course within the scope of the invention. Also, while a description has been made of suitable means for forming the holes or transparent portions 2 and the opaque portion 3, the invention is not limited to these means and a metal sputtering employing a mask, and like methods may also be used as effective means for the purpose.

From the foregoing description it will be seen that in addition to an essential application of an article made by using as its material the optical material of the invention, e.g., an underlaying sheet, ruler, book cover, corner of a book cover, part of a bookmarker, cash card, member's card, commuter's ticket holder, watch band, shoulder belt for bags or tag, the optical material of this invention is not only effective for use as an objective observation optical material in place of sunglasses when its use is required to protect the user from the glare of ultraviolet rays such as the intense sunlight or the reflection from deep snow, but also effective to correct such defects of vision as nearsightedness, farsightedness, astigmatism or presbyopia. These effects are attained by the fact that the shape and size (area) of the plurality of spot-like transparent portions 2, as well as their positions in the opaque portion 3, are specified such that the adjacent transparent portions overlap in the field of view, and there is no break in the field of view thereby providing a continuous field of view. On the other hand, a greater part of the ultraviolet rays in the field of view is blocked by the opaque portion 3 and also the field of view is continuous as mentioned previously. Thus, there is no obstruction from the vision point of view, thus permitting accurate viewing of an object, even in intense ultraviolet rays.

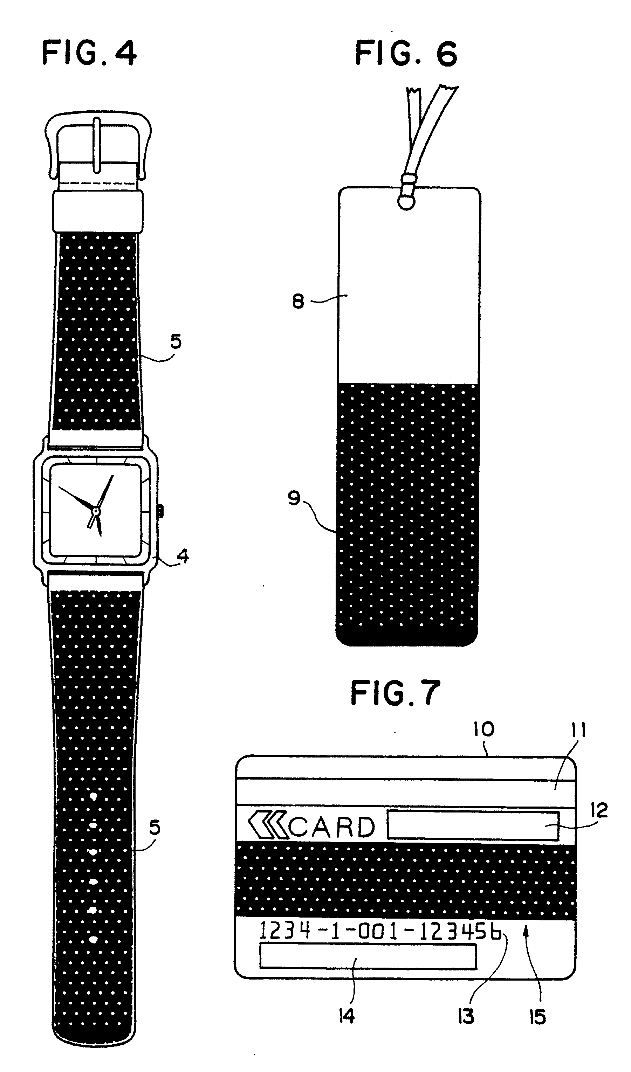

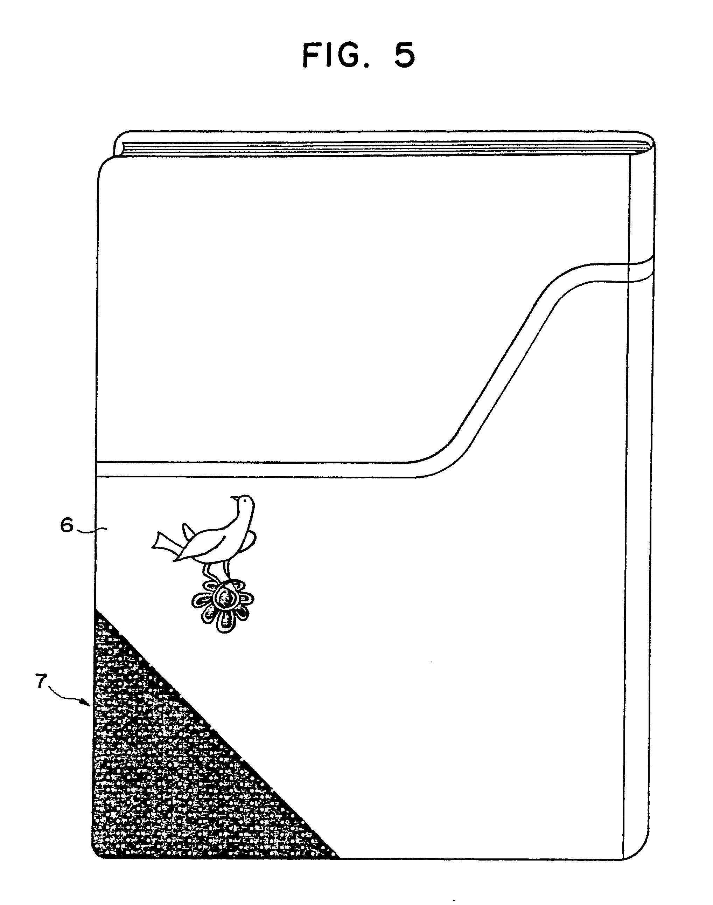

Figure 4 shows by way of example a watch band made of the optical material 1 shown in Figure 1. In the figure, numeral 4 designates the watch proper and 5 the band made of the optical material.

Figure 5 shows an exemplary application in which the optical material 1 of Figure 1 is incorporated in a portion 7 of a book cover 6 and the portion 7 is cut off the cover to read the book through it in place of reading glasses.

Figure 6 also shows an exemplary application in which the optical material 1 of Figure 1 is incorporated in a bookmarker 8 and a portion 9 of the bookmarker 8 is made of the optical material 1.

Figure 7 shows another exemplary application in which the optical material of the invention is incorporated in a bank cash card. In the figure, numeral 10 designates a card, 11 a magnetic recording zone, 12 a bank name stamping portion, 13 a registration number stamping portion, 14 a depositor's name stamping portion, and 15 spot-like transparent portions.

**Claims**

1. An optical sheet material (1) which is capable of permitting sufficient light transmission therethrough to support human vision, at least a part (3) of the sheet being generally opaque but with a large number of spot-like transparent portions (2) distributed uniformly thereover, characterised in that each spot-like transparent portion is formed of substantially hexagonal shape inscribed in or circumscribing a circle having a diameter between 0.7 and 2.0 mm, the sides of the hexagonal shape being curved arcuately inwards, the transparent portions being arranged in a staggered manner such that lines connecting their central points form regular triangles.

2. An optical material as claimed in Claim 1, characterised in that the centres of the transparent portions are spaced at distances in the range from 2.0 to 5.5 mm, and preferably between 3.0 mm and 5.0 mm.

3. An optical material as claimed in any preceding claim, characterised in that each spot-like transparent portion comprises a hole formed through the sheet material.

4. A dual function assembly comprising a readily portable and/or accessible object having a primary function, and also having, as a part of it, or associated with it, a piece of sheet material at least a portion of which has the secondary function of forming an optical device which is capable of permitting sufficient light transmission therethrough to support human vision, at least a part of the sheet being generally opaque but with a large number of spot-like transparent portions distributed uniformly thereover, wherein each spot-like transparent portion is formed of substantially hexagonal shape inscribing or circumscribing a circle having a diameter between 0.7 and 2.0 mm, the sides of the hexagonal shape being curved arcuately inwards, the transparent portions being arranged in a staggered manner such that lines connecting their central points form regular triangles.

5. An assembly as claimed in Claim 4, characterised in that the sheet material is a wrist watch strap, part of a book cover, a bookmark or a credit card.

6. An assembly as claimed in Claim 5, characterised in that the transparent portions are confined to one corner of a book cover.

7. An assembly as claimed in Claim 5, characterised in that the transparent portions are confined to part of a book mark.

8. An assembly as claimed in Claim 5, characterised in that the transparent portions are confined to the central part of a credit card.

9. An assembly as claimed in any of Claims 4 to 8, characterised in that the centres of the transparent portions are spaced at distances in the range from 2.0 to 5.5 mm, and preferably between 3.0 mm and 5.0 mm.

## Revendications

1. Matière optique en feuille (1) qui est capable de permettre une transmission suffisante de lumière à travers elle pour autoriser la vision humaine, au moins une partie (3) de la feuille étant sensiblement opaque, mais comportant un grand nombre d'éléments transparents en forme de taches (2) distribués uniformément sur elle, caractérisée en ce que chaque élément transparent en forme de tache a une forme sensiblement hexagonale inscrite dans ou circonscrivant un cercle ayant un diamètre compris entre 0,7 et 2,0 mm, les côtés de la forme hexagonale étant incurvés en arc vers l'intérieur, les éléments transparents étant disposés de manière échelonnée telle que les lignes reliant leurs points centraux forment des triangles réguliers.

2. Matière optique selon la revendication 1, caractérisée en ce que les centres des éléments transparents sont placés à des distances de l'ordre de 2,0 à 5,5 mm, et de préférence entre 3,0 mm et 5,0 mm.

3. Matière optique selon l'une quelconque des revendications précédentes, caractérisée en ce que chaque élément transparent en forme de tache consiste en un trou formé à travers la matière en feuille.

4. Ensemble à fonction double comprenant un objet facilement portable et/ou accessible ayant une fonction primaire, ainsi qu'un morceau de matière en feuille qui en fait partie ou qui lui est associé et dont au moins une partie a pour fonction secondaire de former un dispositif optique qui est capable de permettre une transmission suffisante de lumière à travers lui pour autoriser la vision humaine, au moins une partie de la feuille étant sensiblement opaque, mais comportant un grand nombre d'éléments transparents en forme de taches qui sont distribuées uniformément sur elle, chaque élément transparent en forme de tache ayant une forme sensiblement hexagonale inscrite dans ou circonscrivant un cercle ayant un diamètre compris entre 0,7 et 2,0 mm, les côtés de la forme hexagonale étant incurvés en arc vers l'intérieur, les éléments transparents étant disposés de manière échelonnée de façon que des lignes qui relient leurs points centraux forment des triangles réguliers.

5. Ensemble selon la revendication 4, caractérisé en ce que la matière en feuille est un bracelet d'une montre bracelet, une partie d'une couverture d'un livre, un signet ou une carte de crédit.

6. Ensemble selon la revendication 5, caractérisé en ce que les éléments transparents sont confinés à un coin d'une couverture de livre.

7. Ensemble selon la revendication 5, caractérisé en ce que les éléments transparents sont confinés à une partie d'un signet.

8. Ensemble selon la revendication 5, caractérisé en ce que les éléments transparents sont confinés à la partie centrale d'une carte de crédit.

9. Ensemble selon l'une quelconque des revendications 4 à 8, caractérisé en ce que les centres des éléments transparents sont placés à des distances de l'ordre de 2,0 à 5,5 mm, et de préférence entre 3,0 mm et 5,0 mm.

## Patentansprüche

1. Blattförmiges optisches Material (1), welches einen ausreichenden Lichtdurchtritt zur Stützung des menschlichen Sehvermögens ermöglicht, mit zumindest einem Teilbereich (3), der zwar im wesentlichen lichtundurchlässig jedoch mit einer großen Anzahl punktartiger transparenter Bereiche (2), die gleichmäßig darauf verteilt sind, versehen ist,

**dadurch gekennzeichnet,**

daß jeder punktartige transparente Bereich eine im wesentlichen hexagonale Kontur aufweist, die einen Innenkreis umschreibt oder von einem Umkreis umschrieben ist mit einem Durchmesser von 0,7 bis 2 mm, die Seiten der hexagonalen Kontur bogenförmig einwärts gekrümmt sind, und die transparenten Bereiche versetzt zueinander angeordnet sind derart, daß die deren Mittelpunkte verbindenden Linien regelmäßige Dreiecke bilden.

2. Blattförmiges optisches Material nach Anspruch 1, dadurch gekennzeichnet, daß die Mittelpunkte der transparenten Bereiche einen Abstand von 2,0 bis 5,5 mm, vorzugsweise zwischen 3,0 und 5,0 mm aufweisen.

3. Blattförmiges optisches Material nach einem der vorhergehenden Ansprüche, dadurch gekennzeichnet, daß jeder punktartige transparente Bereich ein in dem blattförmigen Material ausgebildetes Loch aufweist.

4. Doppelfunktionale Anordnung mit einem leicht bewegbaren und/oder zugänglichen Gegenstand mit einer primären Funktion und mit einem einen Teil des Gegenstandes darstellenden, oder mit diesem verbundenen Teil eines blattförmigen Materials mit zumindest einem Bereich, der die sekundäre Funktion aufweist, eine optische Vorrichtung zu bilden, die einen ausreichenden Lichtdurchtritt zur Stützung des menschlichen Sehvermögens ermöglicht, mit zumindest einem Teilbereich, der zwar im wesentlichen lichtundurchlässig jedoch mit einer großen Anzahl punktartiger transparenter Bereiche, die gleichmäßig darauf verteilt sind, versehen ist, in dem jeder punktartige transparente Bereich eine im wesentlichen hexagonale Kontur aufweist, die einen Innenkreis umschreibt oder von einem Umkreis umschrieben ist mit einem Durchmesser von 0,7 bis 2,0 mm, die Seiten der hexagonalen Kontur bogenförmig einwärts gekrümmt sind, und die transparenten Bereiche versetzt zueinander angeordnet sind derart, daß die deren Mittelpunkt verbindenden Linien regelmäßige Dreiecke bilden.

5. Anordnung nach Anspruch 4, dadurch gekennzeichnet, daß das blattförmige Material ein Armband einer Armbanduhr, ein Teil eines Buchdeckels, ein Lesezeichen oder eine Kreditkarte ist.

6. Anordnung nach Anspruch 5, dadurch gekennzeichnet, daß die transparenten Bereiche auf einen Eckbereich eines Buchdeckels begrenzt sind.

7. Anordnung nach Anspruch 5, dadurch gekennzeichnet, daß die transparenten Bereiche auf einen Teil eines Lesezeichens begrenzt sind.

8. Anordnung nach Anspruch 5, dadurch gekennzeichnet, daß die transparenten Bereiche auf einen mittleren Bereich einer Kreditkarte begrenzt sind.

9. Anordnung nach einem der Ansprüche 4 bis 8, dadurch gekennzeichnet, daß die Mittelpunkte der transparenten Bereiche einen Abstand von 2,0 bis 5,5 mm, vorzugsweise zwischen 3,0 und 5,0 mm, aufweisen.

# FIG. 1

# FIG.2A     FIG.2B     FIG.2C

# FIG. 3

# FIG. 4

# FIG. 6

# FIG. 7

CARD

1234-1-001-123456

# FIG. 5